# EUROPEAN PATENT SPECIFICATION

(11) **EP 3 545 941 B1**
(45) Date of publication and mention of the grant of the patent: **13.10.2021**
(21) Application number: 17873064.4
(22) Date of filing: 22.11.2017
(51) Int. Cl.: A61K 8/31, A61K 8/34, A61K 8/35, A61K 8/81, A61Q 1/02, A61Q 19/00, B05B 5/035, B05B 5/16

(54) **METHOD FOR PRODUCING COATING FILM FOR COSMETIC PREPARATION**
VERFAHREN ZUR HERSTELLUNG EINES BESCHICHTUNGSFILMS FÜR EINE KOSMETISCHE ZUBEREITUNG
PROCÉDÉ DE PRODUCTION D'UN FILM DE REVÊTEMENT D'UNE PRÉPARATION COSMÉTIQUE

(30) Priority: 22.11.2016 JP 2016226824
(43) Date of publication of application: 02.10.2019
(73) Proprietor: Kao Corporation, Chuo-Ku Tokyo 103-8210 (JP)
(72) Inventor: UCHIYAMA, Masayuki, Tokyo 131-0044 (JP); AMARI, Naomi, Haga-gun Tochigi 321-3497 (JP)
(74) Representative: Hoffmann Eitle
(86) International application number: PCT/JP2017/042078
(87) International publication number: WO 2018/097202

(56) References cited:
- WO-A1-2017/069079
- WO-A1-2017/069080
- JP-A- H05 194 145
- JP-A- 2003 506 470
- JP-A- 2003 506 474
- JP-A- 2006 104 211
- JP-A- 2017 078 062
- JP-A- 2017 078 063
- DATABASE GNPD [Online] MINTEL; 18 August 2015 (2015-08-18), anonymous: "Makeup Setting Spray", XP055578800, retrieved from www.gnpd.com Database accession no. 3377203

## Description

### Technical Field

The present invention relates to a method for forming a coating for a cosmetic.

### Background Art

Various methods for forming a coating through electrostatic spraying are known. For example, Patent Literature 1 describes a method for treating skin including electrostatically spraying a composition onto the skin. The composition used in this method contains an electrically insulating liquid material, a conductive substance, a particulate powder material, and a thickener. A cosmetic that contains a pigment, and compositions for skin care are typically used as this composition. Specifically, cosmetic foundation is used as this composition. That is, the invention described in Patent Literature 1 is intended to be mainly used to electrostatically spray the cosmetic foundation and apply the cosmetic foundation on the skin for the purpose of beauty treatment.

Patent Literature 2 describes a disposable cartridge to be used in an electrostatic spraying apparatus for a cosmetic. This electrostatic spraying apparatus is a hand-held and self-contained type. This electrostatic spraying apparatus is used to spray cosmetic foundation as in Patent Literature 1 above.

Mintel online database GNPD, 18.08.2015, "Makeup Setting Spray", accession number 3377203, shows a setting spray for application over makeup.

### Citation List

### Patent Literatures

Patent Literature 1: JP 2006-104211A
Patent Literature 2: JP 2003-507165A (Tokuhyo)

### Summary of Invention

The present invention is directed to a method for forming a coating for a cosmetic, for forming a coating on a surface of skin, including:
a cosmetic applying step of applying a cosmetic containing 10 mass% or more of an oil that is in a solid form at 20°C onto skin; and
an electrostatic spraying step of electrostatically spraying a composition directly onto the skin, thereby forming a coating,
wherein the composition contains a component (a) and a component (b) below:
   (a) one or more volatile substances selected from the group consisting of water, alcohols, and ketones; and
   (b) a polymer having a coating formation ability.

### Brief Description of Drawings

[Fig. 1] Fig. 1 is a schematic diagram showing the configuration of an electrostatic spraying apparatus that is preferably used in the present invention.
[Fig. 2] Fig. 2 is a schematic diagram showing a state in which an electrostatic spraying apparatus is used to perform an electrostatic spraying method.

### Description of Embodiments

When a coating for a cosmetic is formed on skin by performing electrostatic spraying according to the methods described in Patent Literatures 1 and 2, the adhesion between the skin and the coating for a cosmetic formed through electrostatic spraying may be insufficient, and thus the coating for a cosmetic may be damaged or come off due to an external force such as friction.

Incidentally, in the case where a cosmetic containing an oil in a solid form and the like are applied to skin, the oil comes off when friction occurs contact with clothes, skin, or the like, or when the cosmetic comes into contact with water during hand-washing or washing of dishes or the like. Furthermore, although an oil in a solid form is expected to have a good moisturizing performance, the oil is considerably sticky in proportion to the effects, and is unpleasant to use. When a coating for a cosmetic was formed through electrostatic spraying using the methods described in Patent Literatures 1 and 2 in consideration of a case in which a cosmetic containing an oil in a solid form is applied to skin and covered by the coating, the adhesion between the obtained coating for a cosmetic and the skin was insufficient, and there were no improvements in terms of the waterproof properties or the feeling of stickiness.

Thus, the present invention relates to improving a technique regarding an electrostatic spraying method onto skin, and more specifically relates to increasing the adhesion of a coating formed on skin through electrostatic spraying, increasing the waterproof properties of a cosmetic covered by the coating, and forming a non-sticky coating for a cosmetic.

Hereinafter, the present invention will be described based on a preferred embodiment thereof with reference to the drawings.

### Composition that is applied through Electrostatic Spraying Method

In the present invention, a coating for a cosmetic (hereinafter, also referred to as a "coating") is formed by directly applying a composition containing predetermined components onto skin. In the present invention, an electrostatic spraying method is used as a method for forming a coating. The electrostatic spraying method is a method in which a positive or negative high voltage is applied to a composition to electrify the composition, and then the electrified composition is sprayed toward a spray target. The sprayed composition spreads in a space while being repeatedly micronized due to Coulomb repulsion, and during this process or after the composition has attached to the spray target, a solvent that is a volatile substance dries to form a coating on the surface of the spray target.

The composition used in the present invention (this composition is also referred to as "spray composition" hereinafter) is in a liquid form in an environment where the electrostatic spraying method is performed. This composition contains a component (a) and a component (b) below:
(a) one or more volatile substances selected from water, alcohols, and ketones; and
(b) a polymer having a coating formation ability.

Hereinafter, each composition will be described.

A volatile substance to be used as the component (a) is a substance having volatility in a liquid form. The component (a) is blended into the spray composition for the ultimate purpose of forming a dry coating in the following manner: when the spray composition that has been placed in an electric field and sufficiently electrified is discharged toward skin from the tip of a nozzle, the charge density of the spray composition becomes excessive as the component (a) evaporates, and then the component (a) further evaporates while the spray composition is further micronized due to Coulomb repulsion. For this purpose, the vapor pressure of the volatile substance at 20°C is preferably from 0.01 to 106.66 kPa, more preferably from 0.13 to 66.66 kPa, even more preferably from 0.67 to 40.00 kPa, and even more preferably from 1.33 to 40.00 kPa.

Preferable examples of alcohols serving as the volatile substance to be used as the component (a) include chain aliphatic monohydric alcohols, cyclic aliphatic monohydric alcohols, and aromatic monohydric alcohols. Specific examples thereof include ethanol, isopropyl alcohol, butyl alcohol, phenylethyl alcohol, propanol, and pentanol. One or more alcohols selected from these alcohols can be used.

Examples of ketones serving as the conductive substance to be used as the component (a) include acetone, methyl ethyl ketone, and methyl isobutyl ketone. These ketones can be used alone or in combination of two or more.

The volatile substance to be used as the component (a) is more preferably one or more selected from ethanol, isopropyl alcohol, butyl alcohol and water, even more preferably one or more selected from ethanol and butyl alcohol, and even more preferably ethanol.

The spray composition contains, along with the component (a), a polymer having a coating formation ability to be used as the component (b). The polymer having a coating formation ability to be used as the component (b) is commonly a substance that can be dissolved in the volatile substance to be used as the component (a). The term "dissolve" as used herein refers to a state in which a substance is in a dispersed state at 20°C and the dispersion is uniform when visually observed, and preferably transparent or translucent when visually observed.

As the polymer having a coating formation ability, a polymer is used that is appropriate according to the properties of the volatile substance to be used as the component (a). Specifically, polymers having a coating formation ability are roughly classified into water-soluble polymers and water-insoluble polymers. The term "water-soluble polymer" as used herein refers to a polymer having a property in such a manner that when 1 g of the polymer is weighed out and immersed in 10 g of ion-exchanged water in an environment at a pressure of 1 atmosphere and a temperature of 23°C for 24 hours, 0.5 g or more of the immersed polymer dissolves in the water. On the other hand, the term "water-insoluble polymer" as used herein refers to a polymer having a property in such a manner that when 1 g of the polymer is weighed out and immersed in 10 g of ion-exchanged water in an environment at a pressure of 1 atmosphere and a temperature of 23°C for 24 hours, more than 0.5 g of the immersed polymer does not dissolve in the water.

Examples of the water-soluble polymers having a coating formation ability include naturally-occurring macromolecules such as pullulan, hyaluronic acid, chondroitin sulfate, poly-γ-glutamic acid, modified corn starch, β-glucan, glucooligosaccharide, heparin, mucopolysaccharide such as keratosulfate, cellulose, pectin, xylan, lignin, glucomannan, galacturonic acid, psyllium seed gum, tamarind seed gum, gum arabic, gum traganth, water-soluble soybean polysaccharide, alginic acid, carrageenan, laminaran, agar (agarose), fucoidan, methyl cellulose, hydroxypropyl cellulose, and hydroxypropyl methyl cellulose; and synthetic macromolecules such as partially saponified polyvinyl alcohol (when not used in combination with a cross-linking agent), low saponified polyvinyl alcohol, polyvinyl pyrrolidone (PVP), polyethylene oxide, and sodium polyacrylate. These water-soluble polymers can be used alone or in combination of two or more. It is preferable to use pullulan and synthetic macromolecules such as partially saponified polyvinyl alcohol, low saponified polyvinyl alcohol, polyvinyl pyrrolidone, and polyethylene oxide, of these water-soluble polymers, from the viewpoint of easily manufacturing a coating. When polyethylene oxide is used as the water-soluble polymer, its number average molecular weight is preferably from 50,000 to 3,000,000, and more preferably from 100,000 to 2,500,000.

On the other hand, examples of the water-insoluble polymers having a coating formation ability include completely saponified polyvinyl alcohol, which can be insolubilized after the formation of a coating; partially saponified polyvinyl alcohol, which can be cross-linked after the formation of a coating when used in combination with a cross-linking agent; oxazoline modified silicone such as a poly(N-propanoylethyleneimine)-grafted dimethylsiloxane/y-aminopropylmethylsiloxane copolymer; polyvinylacetal diethylamino acetate; zein (main component of corn proteins); polyester; polylactic acid (PLA); an acrylic resin such as a polyacrylonitrile resin and a polymethacrylic acid resin; a polystyrene resin; a polyvinyl butyral resin; a polyethylene terephthalate resin; a polybutylene terephthalate resin; a polyurethane resin; a polyamide resin; a polyimide resin; and a polyamideimide resin. These water-insoluble polymers can be used alone or in combination of two or more. It is preferable to use completely saponified polyvinyl alcohol, which can be insolubilized after the formation of a coating, partially saponified polyvinyl alcohol, which can be cross-linked after the formation of a coating when used in combination with a cross-linking agent, a polyvinyl butyral resin, oxazoline modified silicone such as a poly(N-propanoylethyleneimine)-grafted dimethylsiloxane/γ-aminopropylmethylsiloxane copolymer, water-soluble polyester, zein, and the like, of these water-insoluble polymers.

The content of the component (a) in the spray composition is preferably 50 mass% or more, more preferably 55 mass% or more, and even more preferably 60 mass% or more. In addition, the content is preferably 98 mass% or less, more preferably 96 mass% or less, and even more preferably 94 mass% or less. The content of the component (a) in the spray composition is preferably from 50 to 98 mass%, more preferably from 55 to 96 mass%, and even more preferably from 60 to 94 mass%. When the component (a) is blended into the spray composition in this proportion, the spray composition can sufficiently volatilize when the electrostatic spraying method is performed.

On the other hand, the content of the component (b) in the spray composition is preferably 2 mass% or more, more preferably 4 mass% or more, and even more preferably 6 mass% or more. In addition, the content is preferably 50 mass% or less, more preferably 45 mass% or less, and even more preferably 40 mass% or less. The content of the component (b) in the spray composition is preferably from 2 to 50 mass%, more preferably from 4 to 45 mass%, and even more preferably from 6 to 40 mass%. When the component (b) is blended into the spray composition in this proportion, a desired coating can be successfully formed.

The spray composition may include only the above-described component (a) and component (b) or may include other components in addition to the component (a) and the component (b). Examples of the other components include a plasticizer for the polymer having a coating formation ability to be used as the component (b), a coloring pigment, an extender pigment, a dye, a surfactant, a UV protection agent, a flavoring agent, a repellent, an antioxidant, a stabilizer, an antiseptic, and various vitamins. When the spray composition includes the other components, the blend proportion of the other components is preferably from 0.1 to 30 mass%, and more preferably from 0.5 to 20 mass%.

When the electrostatic spraying method is performed, the viscosity, at 25°C, of the spray composition used in this method is preferably 1 mPa·s or more, more preferably 10 mPa·s or more, and even more preferably 50 mPa·s or more. In addition, the viscosity, at 25°C, is preferably 5,000 mPa·s or less, more preferably 2,000 mPa·s or less, and even more preferably 1,500 mPa·s or less. The viscosity, at 25°C, of the spray composition is preferably from 1 to 5,000 mPa·s, more preferably from 10 to 2,000 mPa·s, and even more preferably from 50 to 1,500 mPa·s. When the spray composition having a viscosity in this range is used, a porous coating, particularly a porous coating composed of a deposit of fibers, can be successfully formed using the electrostatic spraying method. The formation of the porous coating is advantageous from the viewpoint of preventing skin from getting sweaty. The viscosity of the spray composition is measured at 25°C using an E-type viscometer. An E-type viscometer manufactured by Tokyo Keiki Inc. can be used as the E-type viscometer, for example. In this case, a rotor No. 43 can be used as a rotor.

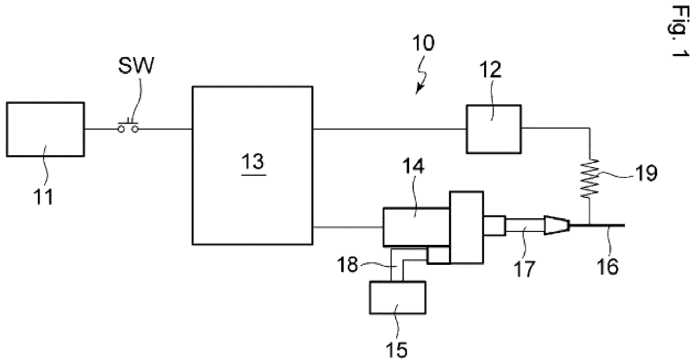

The spray composition is sprayed directly on human skin, which is a spray object, in the electrostatic spraying method. The electrostatic spraying method includes a step of electrostatically spraying the spray composition on the skin using an electrostatic spraying apparatus. FIG. 1 is a schematic diagram illustrating a configuration of an electrostatic spraying apparatus to be preferably used in the present invention. An electrostatic spraying apparatus 10 shown in this diagram includes a low-voltage power source 11. The low-voltage power source 11 can generate a voltage of several volts to a dozen or so volts. It is preferable that the low-voltage power source 11 is constituted by one or more batteries for the purpose of enhancing the portability of the electrostatic spraying apparatus 10. Also, when a battery is used as the low-voltage power source 11, there is an advantage in that the battery can be easily replaced as necessary. An AC adapter or the like can be used as the low-voltage power source 11 instead of the battery.

The electrostatic spraying apparatus 10 also includes a high-voltage power source 12. The high-voltage power source 12 is connected to the low-voltage power source 11 and includes an electric circuit (not shown) that boosts a voltage generated by the low-voltage power source 11 to a high voltage. A voltage boosting electric circuit usually includes a transformer, a capacitor, a semiconductor element, and the like.

The electrostatic spraying apparatus 10 further includes an auxiliary electric circuit 13. The auxiliary electric circuit 13 intervenes between the above-described low-voltage power source 11 and high-voltage power source 12 and has a function of adjusting the voltage of the low-voltage power source 11 to allow the high-voltage power source 12 to stably operate. Furthermore, the auxiliary electric circuit 13 has a function of controlling the rotation rate of a motor provided in a micro gear pump 14, which will be described later. The amount of the spray composition supplied from a container 15 for the spray composition, which will be described later, to the micro gear pump 14 is controlled by controlling the rotation rate of the motor. A switch SW is installed between the auxiliary electric circuit 13 and the low-voltage power source 11, and the operation of the electrostatic spraying apparatus 10 can be started/stopped by turning on/off the switch SW.

The electrostatic spraying apparatus 10 further includes a nozzle 16. The nozzle 16 is made of a conductor including various conductors typified by metal or a non-conductor such as plastic, rubber, or ceramic and has a shape allowing the spray composition to be discharged from the tip of the nozzle. A minute space through which the spray composition flows and that extends in the longitudinal direction of the nozzle 16 is formed inside the nozzle 16. With regard to the size of the cross section of this minute space, the diameter thereof is preferably from 100 to 1,000 µm. The nozzle 16 is in communication with the micro gear pump 14 via a duct 17. The duct 17 may be made of a conductor or a non-conductor. The nozzle 16 is electrically connected to the high-voltage power source 12. This makes it possible to apply a high voltage to the nozzle 16. In this case, in order to prevent a case where excessive current flows when a human body is in direct contact with the nozzle 16, the nozzle 16 is electrically connected to the high-voltage power source 12 via a current limiting resistor 19.

The micro gear pump 14, which is in communication with the nozzle 16 via the duct 17, functions as a supply device for supplying the spray composition accommodated in the container 15 to the nozzle 16. The low-voltage power source 11 supplies power to the micro gear pump 14, so that the micro gear pump 14 operates. The micro gear pump 14 is configured to supply a predetermined amount of the spray composition to the nozzle 16 under the control of the auxiliary electric circuit 13.

The container 15 is connected to the micro gear pump 14 via a flexible duct 18. The spray composition is accommodated in the container 15. It is preferable that the container 15 is of an exchangeable cartridge-type.

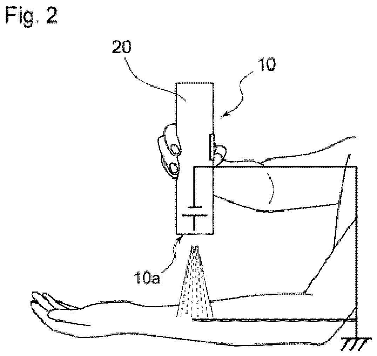

The electrostatic spraying apparatus 10 configured as described above can be used as shown in FIG. 2, for example. FIG. 2 shows the hand-held electrostatic spraying apparatus 10 having dimensions allowing the apparatus to be held by one hand. In the electrostatic spraying apparatus 10 shown in this diagram, all of the members shown in the configuration diagram in FIG. 1 are accommodated in a cylindrical housing 20. The nozzle (not shown) is arranged at one end 10a in the longitudinal direction of the housing 20. The nozzle is arranged in the housing 20 in such a manner that the direction in which the composition is discharged matches the longitudinal direction of the housing 20 and the nozzle projects toward the skin. Since the tip of the nozzle is arranged so as to project toward the skin in the longitudinal direction of the housing 20, the spray composition is less likely to adhere to the housing, and the coating can be stably formed.

When the electrostatic spraying apparatus 10 is operated, a user, that is, a person who forms a coating on his/her skin through electrostatic spraying, holds the apparatus 10 by the hand and directs the one end 10a of the apparatus 10 at which the nozzle (not shown) is arranged toward a portion to be subjected to electrostatic spraying. FIG. 2 shows a state in which the one end 10a of the electrostatic spraying apparatus 10 is directed to the inner side of a forearm of the user. Under these conditions, the apparatus 10 is switched on to perform the electrostatic spraying method. When the apparatus 10 is turned on, an electric field is generated between the nozzle and the skin. In the embodiment shown in FIG. 2, a high positive voltage is applied to the nozzle, and the skin serves as a negative electrode. When the electric field is generated between the nozzle and the skin, the spray composition at the tip of the nozzle is polarized by electrostatic induction, thus shaping the tip of the spray composition into a cone shape. Then, electrified droplets of the spray composition are discharged to the air from the tip of the cone toward the skin along the electric field. When the component (a) used as a solvent evaporates from the electrified spray composition, which has been discharged to the air, the charge density of the surface of the spray composition becomes excessive, and the spray composition spreads in the space while being repeatedly micronized due to Coulomb repulsion and then reaches the skin. In this case, by appropriately adjusting the viscosity of the spray composition, it is possible to cause the sprayed composition to reach the skin in the state in which the composition is in a droplet form. Alternatively, while the composition is being discharged to the space, it is also possible to evaporate the volatile substance used as a solvent from the composition, solidify the polymer having a coating formation ability used as a solute to form fibers while the fibers are stretched and deformed due to an electric potential difference, and deposit the fibers on the surface of the skin. When the viscosity of the spray composition is increased, for example, it is easy to deposit the composition in a fibrous form on the surface of the skin. Accordingly, a porous coating composed of a deposit of fibers is formed on the surface of the skin. The porous coating composed of the deposit of fibers can also be formed by adjusting the distance between the nozzle and the skin, and the voltage applied to the nozzle.

A high electric potential difference is generated between the nozzle and the skin while the electrostatic spraying method is being performed. However, an impedance is very large, and therefore, a current flowing in a human body is extremely small. The inventors of the present invention confirmed that a current flowing in a human body while the electrostatic spraying method is being performed is smaller by several digits than a current flowing in a human body due to static electricity generated in normal life, for example.

When the deposit of fibers is formed using the electrostatic spraying method, the thickness of the fibers expressed as a diameter of a corresponding circle is preferably 10 nm or more, and more preferably 50 nm or more. In addition, the thickness is preferably 3,000 nm or less, and more preferably 1,000 nm or less. The thickness of the fibers can be measured by observing the fibers magnified 10,000 times using a scanning electron microscope (SEM), for example, removing defects (mass of fibers, intersection of fibers, and droplets) from the two-dimensional images of the fibers, selecting any ten fibers, drawing a line orthogonal to the longitudinal direction of each of the fibers, and reading the diameter of the fiber directly.

Although the above-mentioned fiber is a continuous fiber having an infinite length in the formation principle, it is preferable that the fiber has a length at least 100 times longer than its thickness. In this specification, a fiber having a length over 100 times than its thickness is defined as a "continuous fiber". It is preferable that a coating formed using the electrostatic spraying method is a porous discontinuous coating composed of the deposit of continuous fibers. The coating in such a form can be treated as one sheet composed of an aggregate and is characterized by being very soft, and therefore, there is an advantage in that the coating is unlikely to fall apart even when a shearing force is applied to the coating, and the coating has a good property of following to body movement. Also, there is an advantage in that the coating has a good property of diffusing sweat from the skin. Furthermore, there is an advantage in that the coating is easy to take off. In contrast, a continuous coating having no pores is not easy to take off and has a very low property of diffusing sweat, and thus the skin is likely to get sweaty.

The fibrous spray composition reaches the skin in a state in which the composition is electrified. Since the skin is also electrified as described above, the fibers come into intimate contact with the skin due to an electrostatic force. Since there is minute unevenness such as a skin texture on the surface of the skin, an anchor effect is obtained due to the unevenness, and the fibers thus come into further intimate contact with the surface of the skin. After the electrostatic spraying is finished in this manner, the electrostatic spraying apparatus 10 is turned off. Accordingly, the electric field between the nozzle and the skin vanishes, and the electric charge on the surface of the skin is fixed. As a result, the coating exhibits a better adhesion.

Although, as the coating, the porous coating composed of the deposit of fibers has been described above, the form of the coating is not limited thereto. A continuous coating having no pores may be formed, and a porous coating in a form other than the deposit of fibers, for example, a porous coating obtained by forming a plurality of through pores irregularly or regularly in a continuous coating, that is, a discontinuous coating, may be formed. As described above, a coating having a desired shape can be formed by adjusting the viscosity of the spray composition, the distance between the nozzle and the skin, the voltage applied to the nozzle, and the like.

Although the distance between the nozzle and the skin depends on the voltage applied to the nozzle, the distance of 50 to 150 mm is preferable in order to successfully form the coating. The distance between the nozzle and the skin can be measured using a commonly used non-contact sensor or the like.

Regardless of whether or not the coating formed using the electrostatic spraying method is a porous coating, the basis weight of the coating is preferably 0.1 g/m² or more, and more preferably 1 g/m² or more. In addition, the basis weight is preferably 30 g/m² or less, and more preferably 20 g/m² or less. For example, the basis weight of the coating is preferably from 0.1 to 30 g/m², and more preferably from 1 to 20 g/m². Setting the basis weight of the coating in this manner makes it possible to improve the adhesion of the coating. The electrostatic spraying step of electrostatically spraying a composition directly onto skin, thereby forming a coating refers to a step of electrostatically spraying a composition onto skin to which a later-described cosmetic has been applied (may simply be referred to as skin), thereby forming a coating. A step of electrostatically spraying a composition on a region other than skin, thereby forming a sheet composed of fibers, and then applying the sheet to the skin is different from the above-described electrostatic spraying step.

The present invention includes a cosmetic applying step of applying a cosmetic containing 10 mass% or more of an oil that is in a solid form at 20°C onto skin, prior to an electrostatic spraying step of forming the coating through the above-described electrostatic spraying. When a cosmetic is applied prior to the electrostatic spraying step, the surface of the skin is more easily polarized by electrostatic induction. Therefore, the sprayed spray composition is likely to more firmly adhere thereto due to an electrostatic force, and the transparency of the external appearance of the sprayed spray composition can be improved. When the coating is transparent, colored and transparent, or translucent, the skin serving as a foundation is hardly concealed. Preferably, when the coating is a porous coating composed of a deposit of fibers, the adhesion to the skin is high despite being of a high porosity, and a large capillary force is likely to be generated. Furthermore, use of fine fibers makes it easy to increase the specific surface area of the porous coating.

That is to say, the above-described cosmetic applying step is performed using a method other than the electrostatic spraying. When a cosmetic is applied using a method other than the electrostatic spraying prior to the electrostatic spraying step, the above-described effects can be obtained.

When the cosmetic applying step using a cosmetic containing 10 mass% or more of an oil that is in a solid form at 20°C is performed prior to the step of forming a porous coating composed of a deposit of fibers in the electrostatic spraying step, a moisturizing cosmetic holding coating in which a moisturizing cosmetic is present between the fibers included in the porous coating and/or on the surfaces of the fibers is formed. Accordingly, the adhesion of the coating is improved, and the transparency of the coating is maintained or improved. In particular, when the coating is colorless and transparent or translucent, the coating is difficult to visually confirm, and thus can be made to look like a natural skin. When the coating is colored and opaque, the coating has a feeling of transparency and thus can be made to look like part of the skin. It should be noted that "colored" mentioned above includes the color white.

That is to say, the above-described object can be achieved when the cosmetic applying step using a cosmetic containing 10 mass% or more of an oil that is in a solid form at 20°C is performed using a method other than the electrostatic spraying prior to the step of forming a porous coating composed of a deposit of fibers in the electrostatic spraying step.

### A cosmetic that is used in the Cosmetic Applying Step0

In the present invention, the cosmetic that is used in the cosmetic applying step contain 10 mass% or more of an oil that is in a solid form at 20°C. In the present invention, an oil that is in a solid form at 20°C is an oil whose melting peak is observed at 20°C or more using a differential scanning calorimeter (DSC). When a solid sample melts, a thermal energy larger than that of a reference is absorbed as melting heat, and thus a melting peak indicating a change in the status from a solid to a liquid is observed. In the present invention, an oil whose peak is observed at 20°C or more is defined as an oil that is in a solid form at 20°C. Specific measuring methods are as described in Examples.

The adhesion between a coating formed using conventional electrostatic spraying methods and skin may be insufficient, and thus the coating may be damaged or come off due to an external force such as friction. However, the inventors of the present invention found that, if the above-described configuration is used as the electrostatic spraying method and a cosmetic that is applied to skin in advance contain 10 mass% or more of an oil that is in a solid form at 20°C, remarkable effects are achieved in which not only the adhesion of a coating can be increased, but the waterproof properties of a cosmetic covered by the coating can also be increased, and a non-sticky coating for a cosmetic can be formed.

That is to say, the inventors of the present invention found that, if the above-described configuration is used as the electrostatic spraying method and a cosmetic that is applied to skin in advance using a method other than the electrostatic spraying method contains 10 mass% or more of an oil that is in a solid form at 20°C, remarkable effects are achieved in which not only the adhesion of a coating can be increased, but the waterproof properties of a cosmetic covered by the coating can also be increased, and a non-sticky coating for a cosmetic can be formed.

There is no particular limitation on the oil that is in a solid form at 20°C, as long as it is typically used for cosmetics. Examples thereof include a wax, cholesterol derivatives, phytosterol derivatives, dipentaerythritol fatty acid esters, triglycerides, lanolin, lanosterol derivatives, Vaseline, ceramides, a higher alcohol, a higher fatty acid, and the like.

Examples of the wax may include ester waxes such as rice bran wax, carnauba wax, candelilla wax, beeswax, and spermaceti, and hydrocarbon waxes such as ceresin, paraffin wax, microcrystalline wax, polyethylene wax, and polyolefin wax. They may be any of animal waxes, vegetable waxes, mineral waxes, and synthetic waxes.

Examples of the cholesterol derivatives include cholesteryl isostearate, cholesteryl hydroxystearate, cholesteryl macadamiate, and di(cholesteryl/behenyl/octyldodecyl)N-lauroyl-L-glutamate.

Examples of the phytosterol derivatives include di(phytosteryl/behenyl/2-octyldodecyl)N-lauroyl-L-glutamate, di(phytosteryl/2-octyldodecyl)N-lauroyl-L-glutamate, phytosteryl isostearate, and phytosteryl oleate.

Examples of the dipentaerythritol fatty acid esters include dipentaerythritol hexaoxystearate, and dipentaerythritol rosinate.

Examples of the triglycerides include (caprylic/capric/myristic/stearic) triglyceride. These triglycerides may be those partially hydrogenated such as a hardened oil.

Examples of the ceramides include natural ceramide and pseudo-ceramide. Examples of the natural ceramide include ceramides Types1 to 7 in which sphingosine, dihydrosphingosine, phytosphingosine, and sphingadienine are amidated. Examples of the pseudo-ceramide include ceramides described in JP 2013-53146A.

Examples of the higher alcohol include higher alcohols with 12 to 22 carbon atoms. Specific examples thereof include myristyl alcohol, cetyl alcohol, stearyl alcohol, and behenyl alcohol.

Examples of the higher fatty acid include linear fatty acids with 12 to 22 carbon atoms. Examples thereof include lauric acid, myristic acid, palmitic acid, stearic acid, and behenic acid.

Among these oils that are in a solid form at 20°C, at least one selected from among ceramides, higher alcohols, and higher fatty acids is preferable from the viewpoint of improving the durability of the adhesion of a coating and the waterproof properties and the friction resistance of a cosmetic covered by the coating.

The content of the oil that is in a solid form at 20°C in the cosmetic is 10 mass% or more from the viewpoint of improving the durability of the adhesion of a coating and the waterproof properties and the friction resistance of a cosmetic covered by the coating. A preferable content of the oil that is in a solid form at 20°C cannot be unconditionally defined because it varies depending on the purpose of use, but it is preferably more than 10 mass% and 100 mass% or less.

The cosmetic that is used in the present invention may contain as appropriate, in addition to the oil that is in a solid form at 20°C, commonly used components such as water, an oil that is in a liquid form at 20°C, polyols, surfactants, feel modifiers, powders such as coloring pigments, ultraviolet absorbers, thickeners such as water-soluble macromolecules, coating agents, glucoses, metal ion-sequestering agents, lower alcohols, pH control agents, skin conditioning agents, vitamins, antioxidants, antiperspirants, aromatic substances, plant extracts, and antiseptics, within a range where the effects of the present invention are not impaired.

Note that the use applications of these agents are not limited to those of the agents, and may be other use applications depending on the purposes, that is, for example, an antiperspirant may be used as an aromatic substance. Furthermore, the use applications may be combined, that is, for example, an antiperspirant may be used as an agent having the effects of an antiperspirant and an aromatic substance.

The cosmetic preferably contains water from the viewpoint of improving the durability of the adhesion of a coating, the waterproof properties and the friction resistance of a cosmetic covered by the coating, and usability. The content of water in the cosmetic is preferably from 0.1 to 90 mass%, more preferably from 0.5 to 85 mass%, and even more preferably from 1 to 80 mass%.

As described above, the cosmetic may contain an oil that is in a liquid form at 20°C (this oil is also referred to as "liquid oil" hereinafter). Examples of the oil include linear or branched hydrocarbon oils such as liquid paraffin, light isoparaffin, liquid isoparaffin, squalane, and squalene; ester oils such as a plant oil including jojoba oil and olive oil, an animal oil including liquid lanolin, monoalcohol fatty acid ester, and polyhydric alcohol fatty acid ester; and silicone oils such as dimethylpolysiloxane, dimethylcyclopolysiloxane, methylphenylpolysiloxane, methylhydrogenpolysiloxane, and higher alcohol modified organopolysiloxane. Of these, from the viewpoint of usability such as smooth application, the hydrocarbon oils and polar oils such as the ester oils, the plant oils containing a triglyceride etc., and the silicone oils are preferable, and the hydrocarbon oils, the ester oils, and the triglyceride are more preferable. The liquid oils selected from these oils can be used alone or in combination of two or more.

The content of the liquid oil in the cosmetic is preferably 5 mass% or less, more preferably 1 mass% or less, and even more preferably 0.5 mass% or less.

As described above, the cosmetic may contain a polyol. Examples of the polyol include: alkylene glycols such as ethylene glycol, propylene glycol, 1,3-propanediol, and 1,3-butanediol; polyalkylene glycols such as diethylene glycol, dipropylene glycol, polyethylene glycol, and polypropylene glycol; and glycerins such as glycerin, diglycerin, and triglycerin. Of these, from the viewpoint of usability such as smooth application, ethylene glycol, propylene glycol, 1,3-butanediol, dipropylene glycol, polyethylene glycol, glycerin, and diglycerin are preferable, propylene glycol, 1,3-butanediol, and glycerin are more preferable, and propylene glycol and 1,3-butanediol are even more preferable.

The content of the polyol in the cosmetic is preferably from 0.1 to 20 mass%, more preferably from 0.5 to 15 mass%, and even more preferably from 1 to 12 mass%, from the viewpoint of improving the adhesion of a coating, and the aspects of usability such as waterproof properties and non-stickiness of a cosmetic covered by the coating.

As described above, the cosmetic may contain a surfactant. Examples of the surfactant include ionic surfactants and non-ionic surfactants. Examples of the ionic surfactants include anionic surfactants, cationic surfactants, and amphoteric surfactants.

Examples of the anionic surfactants include fatty acids with 12 to 22 carbon atoms and salts thereof, such as sodium laurate and arginine stearate; alkyl sulfuric acid esters with 12 to 22 carbon atoms and salts thereof, such as sodium lauryl sulfate and potassium lauryl sulfate; alkyl ether sulfuric acid esters with 12 to 22 carbon atoms and salts thereof, such as triethanolamine polyoxyethylene lauryl sulfate; N-acyl sarcosines with 12 to 22 carbon atoms and salts thereof, such as sodium lauroyl sarcosinate; alkyl phosphates with 12 to 22 carbon atoms and salts thereof, such as sodium monostearyl phosphate; polyoxyethylene alkyl ether phosphoric acids with 12 to 22 carbon atoms and salts thereof, such as sodium polyoxyethylene oleyl ether phosphate and sodium polyoxyethylene stearyl ether phosphate; dialkyl sulfosuccinic acid with 12 to 24 carbon atoms and salts thereof, such as di-2-ethylhexyl sodium sulfosuccinate; N-alkyloylmethyl taurines with 12 to 22 carbon atoms and salts thereof, such as sodium N-stearoyl-N-methyltaurate; and N-acyl glutamic acids with 12 to 22 carbon atoms and salts thereof, such as sodium dilauroyl glutamate, monosodium N-lauroyl glutamate, sodium N-stearoyl-L-glutamate, arginine N-stearoyl-L-glutamate, sodium N-stearoyl glutamate, and sodium N-myristoyl-L-glutamate.

The cationic surfactants are preferably quaternary ammonium salts or the like, and examples thereof include alkyltrimethylammonium chlorides such as stearyltrimethylammonium chloride and lauryltrimethylammonium chloride, dialkyldimethyl ammonium chloride, trialkylmethylammonium chloride, alkylamine salt, and the like. Examples of the amphoteric surfactants include alkyl dimethyl amine oxide, alkyl carboxybetaine, alkyl sulfobetaine, amide amino acid salt, and alkyl amide propyl betaine, and alkyl amide propyl betaine is preferable.

Examples of the non-ionic surfactants include polyglycerin fatty acid ester, propylene glycol fatty acid ester, pentaerythritol fatty acid ester, polyoxyethylene sorbitan fatty acid ester, polyoxyethylene sorbit fatty acid ester, polyoxyethylene glycerin fatty acid ester, polyethylene glycol fatty acid ester, polyoxyethylene alkyl ether, polyoxyethylene phytosterol, polyoxyethylene phytostanol, polyoxyethylene polyoxypropylene alkyl ether, polyoxyethylene alkylphenyl ether, polyoxyethylene castor oil, polyoxyethylene hardened castor oil, polyoxyethylene lanolin, polyoxyethylene lanolin alcohol, polyoxyethylene beeswax derivative, polyoxyethylene alkylamine, polyoxyethylene fatty acid amide, polyoxyethylene alkylphenyl formaldehyde condensate, homogeneous polyoxyethylene alkyl ether, and polyether modified silicone.

The surfactants are preferably ionic surfactants, and more preferably anionic surfactants, from the viewpoint of improving emulsion stability. Furthermore, the anionic surfactants are preferably one or more selected from polyoxyethylene alkyl ether phosphoric acids with 12 to 22 carbon atoms and salts thereof, N-alkyloylmethyl taurine sodium with 12 to 22 carbon atoms, and N-acyl glutamate with 12 to 22 carbon atoms, and from the viewpoint of usability, they are more preferably N-alkyloylmethyl taurine sodium with 12 to 22 carbon atoms. N-alkyloylmethyl taurine sodium with 12 to 22 carbon atoms has preferably 12 to 20 carbon atoms, and more preferably 14 to 20 carbon atoms, and, specifically, it is more preferably N-stearoyl-N-methyl taurine sodium.

The surfactants can be used alone or in combination of two or more. The content of the surfactant in the cosmetic is preferably 0.01 mass% or more, more preferably 0.05 mass% or more, and even more preferably 0.1 mass% or more, and preferably 5 mass% or less, and more preferably 4 mass% or less, from the viewpoint of stably disperse the components. In addition, the content is more preferably 3 mass% or less, and even more preferably 2.5 mass% or less, from the viewpoint of reducing stickiness. The content of the surfactant is the content of compounds excluding counter ions.

As described above, the cosmetic may contain a feel modifier. Examples of the feel modifier include a silicone oil. Examples of the silicone oil include dimethylpolysiloxane, dimethylcyclopolysiloxane, methylphenylpolysiloxane, methylhydrogenpolysiloxane, and higher alcohol modified organopolysiloxane. From the viewpoint of causing a coating formed using the electrostatic spraying method to adhere to skin, the silicone oil preferably contains dimethylpolysiloxane.

The content of the silicone oil in the cosmetic is preferably 10 mass% or less, more preferably 5 mass% or less, even more preferably 1 mass% or less, and even more preferably 0.1 mass% or less, from the viewpoint of the adhesion to skin.

The kinetic viscosity at 25°C of the silicone oil is preferably 3 mm²/s or more, more preferably 4 mm²/s or more, and even more preferably 5 mm²/s or more, and preferably 30 mm²/s or less, more preferably 20 mm²/s or less, and even more preferably 10 mm²/s or less, from the viewpoint of causing a coating formed using the electrostatic spraying method to adhere to skin.

As described above, the cosmetic may contain a coloring pigment. The content of coloring pigment in the cosmetic is preferably less than 0.1 mass%, more preferably 0.05 mass% or less, even more preferably 0.01 mass% or less, and even more preferably 0.001 mass% or less, from the viewpoint of improving the adhesion between a coating formed using the electrostatic spraying method and skin. In the present invention, the coloring pigment refers to a pigment not containing a transparent pigment, and a white pigment is included in the coloring pigment.

The cosmetic containing 10 mass% or more of an oil that is in a solid form at 20°C can be applied on the skin with various methods. For example, a thin layer of the cosmetic can be formed by applying the cosmetic on the skin with a dripping method, a sprinkling method, or the like, and performing a step of spreading the cosmetic to cause the cosmetic to bond with the skin. In the step of spreading the cosmetic, a method such as smearing using the user's own finger, a tool such as an applicator, or the like can be applied, for example. Although it is sufficient that the cosmetic is merely dripped or sprinkled, performing the spreading step makes it possible to cause the cosmetic to bond with the skin, thus making it possible to sufficiently improve the adhesion of the coating. As another method, a thin layer of the cosmetic can be formed by spraying the cosmetic on the skin. In this case, it is not particularly necessary to separately spread the cosmetic, but a spreading operation may be performed after spraying.

That is to say, the cosmetic containing 10 mass% or more of an oil that is in a solid form at 20°C can be applied on the skin with various methods, using a method other than the electrostatic spraying. For example, a thin layer of the cosmetic can be formed by applying the cosmetic on the skin with a dripping method, a sprinkling method, or the like, and performing a step of spreading the cosmetic and forming a coating film to cause the cosmetic to bond with the skin. Although it is sufficient that the cosmetic is merely dripped or sprinkled, performing the step of spreading the cosmetic and forming a coating film makes it possible to cause the cosmetic to bond with the skin, thus making it possible to sufficiently improve the adhesion of the coating. It is also possible to form a coating film by directly applying the cosmetic onto skin, without using the dripping method or the sprinkling method. Regardless of whether or not the dripping method or the sprinkling method is performed, the step of applying the cosmetic and forming a coating film can be performed, for example, using a method such as smearing using the user's own finger, a tool such as an applicator, or the like.

It is sufficient that the amount of the cosmetic that is applied on skin is set to be a necessary and sufficient amount with which the adhesion between the skin and the coating is improved. The amount of the cosmetic that is applied on skin is set in such a manner that the basis weight of the cosmetic is preferably 0.1 g/m² or more, and more preferably 0.2 g/m² or more, and preferably 40 g/m² or less, and more preferably 35 g/m² or less. The amount of the cosmetic that is applied on skin is set in such a manner that the basis weight of the cosmetic is preferably from 0.1 to 40 g/m², and more preferably from 0.2 to 35 g/m².

Furthermore, the amount of the cosmetic that is applied on skin is preferably 5 g/m² or more, more preferably 10 g/m² or more, and even more preferably 15 g/m² or more, and preferably 50 g/m² or less, and more preferably 45 g/m² or less, from the viewpoint of improving the adhesion between skin and a coating, and improving transparency.

Furthermore, it is also possible to apply a cosmetic other than the above-described cosmetic onto skin, prior to or subsequent to applying the above-described cosmetic onto the skin.

The method for forming a coating for a cosmetic as described above is useful as various beauty treatment methods that are not intended to be used as a method of operation, treatment, or diagnosis of a human body. For the purpose of beauty treatment, the coating formation method according to the present invention can be applied to whitening of skin, concealment of specks on skin, concealment of dullness/dark areas of skin, concealment of wrinkles of skin, shading of skin, protection of skin from ultraviolet rays, and moisturization of skin, for example.

Although the present invention has been described based on the preferred embodiment above, the present invention is not limited to the above-mentioned embodiment. In the above-mentioned embodiment, a person who wants to form a coating on his/her skin holds the electrostatic spraying apparatus 10 and generates an electric field between the nozzle of the apparatus 10 and his/her skin, for example. However, a person who wants to form a coating on his/her skin need not hold the electrostatic spraying apparatus 10 as long as an electric field is generated between the nozzle and the skin.

With respect to the above-described embodiment, the present invention further discloses the following aspects of the coating formation method.
<1> A method for forming a coating for a cosmetic on a surface of skin, including:
   a cosmetic applying step of applying a solid cosmetic containing 10 mass% or more of an oil that is in a solid form at 20°C onto skin; and an electrostatic spraying step of electrostatically spraying a composition directly onto the skin, thereby forming a coating,
   wherein the composition contains a component (a) and a component (b) below:
      (a) one or more of volatile substances selected from the group consisting of water, alcohols, and ketones; and
      (b) a polymer having a coating formation ability.
<2> A method for forming a coating for a cosmetic on a surface of skin, including:
   a cosmetic applying step of applying a solid cosmetic containing 10 mass% or more of an oil, that is in a solid form at 20°C, onto skin using a method other than electrostatic spraying; and an electrostatic spraying step of electrostatically spraying a composition directly onto the skin, thereby forming a coating,
   wherein the composition contains a component (a) and a component (b) below:
      (a) one or more of volatile substances selected from the group consisting of water, alcohols, and ketones; and
      (b) a polymer having a coating formation ability.
<3> The method for forming a coating for a cosmetic as set forth in clause <1> or <2>, wherein in the electrostatic spraying step, the composition is electrostatically sprayed on the skin to form a porous coating.
<4> The method for forming a coating for a cosmetic as set forth in any one of clauses <1> to <3>, wherein in the electrostatic spraying step, an electrostatic spraying apparatus is used to electrostatically spray the composition on the skin to form a porous coating composed of a deposit of fibers, and
   the electrostatic spraying apparatus includes:
   a container in which the composition is accommodated;
   a nozzle from which the composition is discharged;
   a supply device that supplies the composition accommodated in the container to the nozzle; and
   a power source that applies a voltage to the nozzle.
<5> The method for forming a coating for a cosmetic as set forth in any one of clauses <1> to <4>, wherein the vapor pressure of the volatile substance (a) at 20°C is preferably from 0.01 to 106.66 kPa, more preferably from 0.13 to 66.66 kPa, even more preferably from 0.67 to 40.00 kPa, and even more preferably from 1.33 to 40.00 kPa.
<6> The method for forming a coating for a cosmetic as set forth in any one of clauses <1> to <5>, wherein the volatile substance (a) is alcohol, the alcohol is chain aliphatic monohydric alcohols, cyclic aliphatic monohydric alcohols, and preferably is aromatic monohydric alcohols, and these alcohols can be used alone or in combination of two or more, and
   the alcohol is particularly preferably ethanol, isopropyl alcohol, butyl alcohol, phenylethyl alcohol, propanol, and pentanol.
<7> The method for forming a coating for a cosmetic as set forth in any one of clauses <1> to <6>, wherein the volatile substance (a) is one or more selected from ethanol, isopropyl alcohol, butyl alcohol, and water, preferably one or two selected from ethanol and butyl alcohol, and more preferably ethanol.
<8> The method for forming a coating for a cosmetic as set forth in any one of clauses <1> to <7>, wherein the polymer (b) having a coating formation ability is a substance that can be dissolved in the volatile substance (a), and includes water-soluble polymers and water-insoluble polymers, and
   the term "dissolve" refers to a state in which a substance is in a dispersed state at 20°C and the dispersion is uniform when visually observed, and preferably transparent or translucent when visually observed.
<9> The method for forming a coating for a cosmetic as set forth in any one of clauses <1> to <8>, wherein the water-soluble polymers having a coating formation ability are one or more water-soluble macromolecules selected from: naturally-occurring polymers such as pullulan, hyaluronic acid, chondroitin sulfate, poly-γ-glutamic acid, modified corn starch, β-glucan, glucooligosaccharide, heparin, mucopolysaccharide such as keratosulfate, cellulose, pectin, xylan, lignin, glucomannan, galacturonic acid, psyllium seed gum, tamarind seed gum, gum arabic, gum traganth, water-soluble soybean polysaccharide, alginic acid, carrageenan, laminaran, agar (agarose), fucoidan, methyl cellulose, hydroxypropyl cellulose, and hydroxypropyl methyl cellulose; partially saponified polyvinyl alcohol (when not used in combination with a cross-linking agent); low saponified polyvinyl alcohol; polyvinyl pyrrolidone (PVP); polyethylene oxide; and sodium polyacrylate, and preferably one or more water-soluble macromolecules selected from pullulan, partially saponified polyvinyl alcohol, low saponified polyvinyl alcohol, polyvinyl pyrrolidone, and polyethylene oxide.
<10> The method for forming a coating for a cosmetic as set forth in any one of clauses <1> to <9>, wherein the water-insoluble polymers having a coating formation ability are one or more water-insoluble polymers selected from completely saponified polyvinyl alcohol, which can be insolubilized after the formation of a coating; partially saponified polyvinyl alcohol, which can be cross-linked after the formation of a coating when used in combination with a cross-linking agent; oxazoline modified silicone such as a poly(N-propanoylethyleneimine)-grafted dimethylsiloxane/y-aminopropylmethylsiloxane copolymer; polyvinylacetal diethylamino acetate; zein (main component of corn proteins); polyester; polylactic acid (PLA); an acrylic resin such as a polyacrylonitrile resin or a polymethacrylic acid resin; a polystyrene resin; a polyvinyl butyral resin; a polyethylene terephthalate resin; a polybutylene terephthalate resin; a polyurethane resin; a polyamide resin; a polyimide resin; and a polyamideimide resin, and preferably one or more water-insoluble polymers selected from completely saponified polyvinyl alcohol, which can be insolubilized after the formation of a coating, partially saponified polyvinyl alcohol, which can be cross-linked after the formation of a coating when used in combination with a cross-linking agent, a polyvinyl butyral resin, oxazoline modified silicone such as a poly(N-propanoylethyleneimine)-grafted dimethylsiloxane/y-aminopropylmethylsiloxane copolymer, water-soluble polyester, and zein.
<11> The method for forming a coating for a cosmetic as set forth in any one of clauses <1> to <10>, wherein the content of the component (a) in the composition is preferably 50 mass% or more, more preferably 55 mass% or more, and even more preferably 60 mass% or more, and preferably 98 mass% or less, more preferably 96 mass% or less, and even more preferably 94 mass% or less, and the content of the component (a) in the composition is preferably from 50 to 98 mass%, more preferably from 55 to 96 mass%, and even more preferably from 60 to 94 mass%.
<12> The method for forming a coating for a cosmetic as set forth in any one of clauses <1> to <11>, wherein the content of the component (b) in the composition is preferably 2 mass% or more, more preferably 4 mass% or more, and even more preferably 6 mass% or more, and preferably 50 mass% or less, more preferably 45 mass% or less, and even more preferably 40 mass% or less, and the content of the component (b) in the composition is preferably from 2 to 50 mass%, more preferably from 4 to 45 mass%, and even more preferably from 6 to 40 mass%.
<13> The method for forming a coating for a cosmetic as set forth in any one of clauses <1> to <12>, wherein the composition includes only the component (a) and component (b) or includes other components in addition to the component (a) and the component (b), and
   the other components include a plasticizer for the polymer (b) having a coating formation ability, a coloring pigment, an extender pigment, a dye, a surfactant, a UV protection agent, a flavoring agent, a repellent, an antioxidant, a stabilizer, an antiseptic, and various vitamins.
<14> The method for forming a coating for a cosmetic as set forth in clause <13>, wherein when the composition includes the other components, the blend proportion of the other components is preferably from 0.1 to 30 mass%, and more preferably from 0.5 to 20 mass%.
<15> The method for forming a coating for a cosmetic as set forth in any one of clauses <1> to <14>, wherein the viscosity of the composition at 25°C is preferably 1 mPa·s or more, more preferably 10 mPa·s or more, and even more preferably 50 mPa·s or more, and preferably 5,000 mPa·s or less, more preferably 2,000 mPa·s or less, and even more preferably 1,500 mPa·s or less, and the viscosity of the composition at 25°C is preferably from 1 to 5,000 mPa·s, more preferably from 10 to 2,000 mPa·s, and even more preferably from 50 to 1,500 mPa·s.
<16> The method for forming a coating for a cosmetic as set forth in any one of clauses <1> to <15>, wherein the electrostatic spraying method is performed using an electrostatic spraying apparatus,
   the electrostatic spraying apparatus includes a nozzle, and
   the nozzle is made of a conductor including various conductors typified by metal or a non-conductor such as plastic, rubber, or ceramic and has a shape allowing the composition to be discharged from the tip of the nozzle.
<17> The method for forming a coating for a cosmetic as set forth in any one of clauses <1> to <16>, wherein the electrostatic spraying method is performed using an electrostatic spraying apparatus,
   the electrostatic spraying apparatus includes a nozzle and a housing,
   the nozzle is arranged at one end in a longitudinal direction of the housing, and
   the nozzle is arranged in the housing in such a manner that the direction in which the composition is discharged matches the longitudinal direction of the housing and the nozzle projects toward the skin.
<18> The method for forming a coating for a cosmetic as set forth in any one of clauses <1> to <17>, comprising stretching and deforming the jetted sprayed composition by an electric potential difference to form fibers while evaporating the volatile substance serving as a solvent in the sprayed composition from droplets and solidifying the polymer having a coating formation ability and serving as a solute.
<19> The method for forming a coating for a cosmetic as set forth in any one of clauses <1> to <18>, wherein the electrostatic spraying method is performed using an electrostatic spraying apparatus,
   the electrostatic spraying apparatus includes a nozzle, and
   the distance between the nozzle and the skin is set to be from 50 to 150 mm.
<20> The method for forming a coating for a cosmetic as set forth in any one of clauses <1> to <19>, wherein the basis weight of the coating formed using the electrostatic spraying method is preferably 0.1 g/m² or more, and more preferably 1 g/m² or more, and preferably 30 g/m² or less, and more preferably 20 g/m² or less, and the basis weight of the coating is preferably from 0.1 to 30 g/m², and more preferably from 1 to 20 g/m².
<21> The method for forming a coating for a cosmetic as set forth in any one of clauses <1> to <20>, wherein the oil that is in a solid form at 20°C is a wax, cholesterol derivatives, phytosterol derivatives, dipentaerythritol fatty acid esters, triglycerides, lanolin, lanosterol derivatives, Vaseline, ceramides, a higher alcohol, or a higher fatty acid.
<22> The method for forming a coating for a cosmetic as set forth in any one of clauses <1> to <21>, wherein the content of the oil that is in a solid form at 20°C in the cosmetic is preferably more than 10 mass% and 100 mass% or less.
<23> The method for forming a coating for a cosmetic as set forth in any one of clauses <1> to <22>, wherein the cosmetic contains, in addition to the oil that is in a solid form at 20°C, water, an oil that is in a liquid form at 20°C, a polyol, a surfactant, a feel modifier, a powder such as a coloring pigment, an ultraviolet absorber, a thickener such as a water-soluble macromolecule, a coating agent, a glucose, a metal ion-sequestering agent, a lower alcohol, a pH control agent, a skin conditioning agent, vitamins, an antioxidant, an antiperspirant, an aromatic substance, a plant extract, or an antiseptic.
<24> The method for forming a coating for a cosmetic as set forth in any one of clauses <1> to <23>, wherein the cosmetic contains water, and the content of water in the cosmetic is preferably from 0.1 to 90 mass%, more preferably from 0.5 to 85 mass%, and even more preferably from 1 to 80 mass%.
<25> The method for forming a coating for a cosmetic as set forth in any one of clauses <1> to <24>,
   wherein the cosmetic contains an oil that is in a liquid form at 20°C, and
   the oil that is in a liquid form at 20°C is linear or branched hydrocarbon oils such as liquid paraffin, light isoparaffin, liquid isoparaffin, squalane, or squalene; ester oils such as a plant oil including jojoba oil or olive oil, an animal oil including liquid lanolin, monoalcohol fatty acid ester, or polyhydric alcohol fatty acid ester; or silicone oils such as dimethylpolysiloxane, dimethylcyclopolysiloxane, methylphenylpolysiloxane, methylhydrogenpolysiloxane, or higher alcohol modified organopolysiloxane.
<26> The method for forming a coating for a cosmetic as set forth in any one of clauses <1> to <25>, wherein the content of the oil that is in a liquid form at 20°C in the cosmetic is 5 mass% or less, more preferably 1 mass% or less, and even more preferably 0.5 mass% or less.
<27> The method for forming a coating for a cosmetic as set forth in any one of clauses <1> to <26>,
   wherein the cosmetic contains a polyol, and
   the polyol is alkylene glycols such as ethylene glycol, propylene glycol, 1,3-propanediol, or 1,3-butanediol; polyalkylene glycols such as diethylene glycol, dipropylene glycol, polyethylene glycol, or polypropylene glycol; or glycerins such as glycerin, diglycerin, or triglycerin.
<28> The method for forming a coating for a cosmetic as set forth in any one of clauses <1> to <27>, wherein the content of the polyol in the cosmetic is preferably from 0.1 to 20 mass%, more preferably from 0.5 to 15 mass%, and even more preferably from 1 to 12 mass%.
<29> The method for forming a coating for a cosmetic as set forth in any one of clauses <1> to <28>,
   wherein the cosmetic contains a surfactant,
   the surfactant is an ionic surfactant or a non-ionic surfactant, and
   the ionic surfactant is an anionic surfactant, a cationic surfactant, or an amphoteric surfactant.
<30> The method for forming a coating for a cosmetic as set forth in clause <29>,
   wherein the surfactant is an ionic surfactant,
   the ionic surfactant is an anionic surfactant, and
   the anionic surfactant is one or more selected from polyoxyethylene alkyl ether phosphoric acids with 12 to 22 carbon atoms and salts thereof, N-alkyloylmethyl taurine sodium with 12 to 22 carbon atoms, and N-acyl glutamate with 12 to 22 carbon atoms.
<31> The method for forming a coating for a cosmetic as set forth in clause <29> or <30>, wherein surfactants can be used alone or in combination of two or more, and the content of the surfactant in the cosmetic expressed as a content of compounds excluding counter ions is preferably 0.01 mass% or more, more preferably 0.05 mass% or more, and even more preferably 0.1 mass% or more, preferably 5 mass% or less, more preferably 4 mass% or less, even more preferably 3 mass% or less, and even more preferably 2.5 mass% or less.
<32> The method for forming a coating for a cosmetic as set forth in any one of clauses <1> to <31>,
   wherein the cosmetic contains a feel modifier,
   the feel modifier is a silicone oil, and
   the silicone oil is dimethylpolysiloxane, dimethylcyclopolysiloxane, methylphenylpolysiloxane, methylhydrogenpolysiloxane, or higher alcohol modified organopolysiloxane.
<33> The method for forming a coating for a cosmetic as set forth in clause <32>,
   wherein the content of the silicone oil in the cosmetic is 10 mass% or less, more preferably 5 mass% or less, even more preferably 1 mass% or less, and even more preferably 0.1 mass% or less, and
   the kinetic viscosity, at 25°C, of the silicone oil is preferably 3 mm²/s or more, more preferably 4 mm²/s or more, and even more preferably 5 mm²/s or more, and preferably 30 mm²/s or less, more preferably 20 mm²/s or less, and even more preferably 10 mm²/s or less.
<34> The method for forming a coating for a cosmetic as set forth in any one of clauses <1> to <33>,
   wherein the cosmetic contains a coloring pigment, and
   the content of the coloring pigment in the cosmetic is preferably less than 0.1 mass%, more preferably 0.05 mass% or less, even more preferably 0.01 mass% or less, and even more preferably 0.001 mass% or less.
<35> The method for forming a coating for a cosmetic as set forth in any one of clauses <1> to <34>, wherein the amount of the cosmetic that is applied on skin is set in such a manner that the basis weight of the cosmetic is preferably 0.1 g/m² or more, and more preferably 0.2 g/m² or more, and preferably 40 g/m² or less, and more preferably 35 g/m² or less,
   the amount of the cosmetic that is applied on skin is set in such a manner that the basis weight of the cosmetic is preferably from 0.1 to 40 g/m², and more preferably from 0.2 to 35 g/m², and
   the amount of the cosmetic that is applied on skin is preferably 5 g/m² or more, more preferably 10 g/m² or more, and even more preferably 15 g/m² or more, preferably 50 g/m² or less, and more preferably 45 g/m² or less.
<36> The method for forming a coating for a cosmetic as set forth in any one of clauses <1> to <35>, wherein prior to or subsequent to applying the above-described cosmetic onto skin, a cosmetic other than the above-described cosmetic are applied onto skin.
<37> The method for forming a coating for a cosmetic as set forth in any one of clauses <1> to <36>, wherein in the cosmetic applying step, the cosmetic is dabbed onto the skin.

### Examples

Hereinafter, the present invention will be described more specifically by way of examples. However, the scope of the present invention is not limited to these examples. Unless otherwise stated, "%" means "mass%".

### Measurement of Melting Peak Temperature

10 mg of sample was put into an aluminum sample pan for thermal analysis, and a difference in the amount of heat between a measurement sample and a reference (empty sample tube) was measured using a differential scanning calorimeter (DSC; manufactured by Mettler Toledo). The measurement was performed while increasing the temperature from 0°C to 150°C at 1°C/min, and the melting peak temperature was measured.

### Example 1

### (1) Preparation of spray composition

99.5% Ethanol and 1-butanol were used as the component (a) of the spray composition. Polyacrylic acid was used as the component (b). The blend proportions of ethanol and 1-butanol used as the component (a) were 52% and 26%, respectively, and the blend proportion of the component (b) was 22%.

### (2) Preparation of cosmetic to be used in cosmetic applying step

55 mg of cosmetic product 6 was used as the cosmetic. Table 1 below shows the composition of the cosmetic product 6.

### (3) Cosmetic applying step

The above-mentioned cosmetic product 6 was dripped on the inner side of a forearm of a human, spread so as to have an area having a diameter of 4 cm or more and less than 6 cm using a finger, and allowed to bond therewith, and a thin layer was thus formed. The amount of the dripped cosmetic product 6 was 55 mg, and that amount was in such a manner that the presence of the cosmetic product 6 could be confirmed by visual observation or by touch due to the region in which the cosmetic product 6 was spread being wet or moist, or having a different texture, or the like. As a result, the amount of the cosmetic product 6 applied on the skin was set in such a manner that the basis weight of the cosmetic product 6 was 28.0 g/m² and the total basis weight of the cosmetic was 0.25 g/m².

### (4) Electrostatic spraying step

The electrostatic spraying method was performed for 20 seconds toward the thin layer formed in the cosmetic applying step of (3) using the electrostatic spraying apparatus 10 having the configuration shown in FIG. 1 and the external appearance shown in FIG. 2. The electrostatic spraying method was performed under the conditions described below.
- Applied voltage: 10 kV
- Distance between nozzle and skin: 100 mm
- Discharge amount of spray composition: 5 ml/h
- Environment: 25°C, 30%RH

A porous coating composed of a deposit of fibers was formed on the surface of the skin through the electrostatic spraying. The coating had a circular shape having a diameter of about 4 cm, and had a mass of about 5.5 mg. The thickness of the fibers measured with the above-described method was 506 nm.

### Examples 2 and 5 to 12

A coating was formed as in Example 1, except that the spray composition and the cosmetic product in Example 1 were changed to those shown in Table 2. Table 1 below shows the composition of cosmetic products 1 to 7.

### Examples 3 and 4

A coating was formed as in Example 2, except that the spray composition in Example 2 was changed to those shown in Table 2.

### Comparative Examples 1 to 4

These comparative examples are examples in which the cosmetic applying step was not performed in Examples 1 to 4. A porous coating composed of a deposit of fibers was obtained as in Example 1, except for this aspect.

### Comparative Examples 5 and 6

These comparative examples are examples in which only a cosmetic product was applied in Examples 5 and 8. In these comparative examples, formation of a coating using the electrostatic spraying method was not performed.

### Comparative Examples 7 and 8

### (1) Preparation of spray composition

99.5% Ethanol was used as the component (a) of the spray composition. Polyvinyl butyral was used as the component (b). As other components, di(phytosteryl/octyl dodecyl)lauroyl glutamate was used. The blend proportion of ethanol as the component (a) was 80%, that of polyvinyl butyral as the component (b) was 14%, and that of di(phytosteryl/octyl dodecyl)lauroyl glutamate as the other components was 6%.

### (2) Formation of sheet in the electrostatic spraying step

The electrostatic spraying method was performed for 20 seconds onto a stainless steel plate, using the electrostatic spraying apparatus 10 having the configuration shown in FIG. 1 and the external appearance shown in FIG. 2. The electrostatic spraying method was performed under the conditions described below.
- Applied voltage: 10 kV
- Distance between nozzle and skin: 100 mm
- Discharge amount of spray composition: 5 ml/h
- Environment: 25°C, 30%RH

A porous coating composed of a deposit of fibers was formed on the surface of the stainless steel plate through the electrostatic spraying. The coating had a circular shape with a diameter of about 4 cm, and had a mass of about 5.5 mg. The thickness of the fibers measured using the above-described method was 506 nm.

### (3) Cosmetic applying step

The above-mentioned cosmetic product 1 or 4 was dripped on the inner side of a forearm of a human, spread so as to have an area having a diameter of 4 cm or more and less than 6 cm using a finger, and allowed to bond therewith, and a thin layer was thus formed. The amount of the dripped cosmetic product 1 or 4 was 55 mg, and that amount was in such a manner that the presence of the cosmetic product 1 or 4 could be confirmed by visual observation or by touch due to the region in which the cosmetic product 1 or 4 was spread being wet or moist, or having a different texture, or the like. As a result, the amount of the cosmetic product 1 or 4 applied on the skin was set in such a manner that the basis weight of the cosmetic product 1 or 4 was 28.0 g/m² and the total basis weight of the cosmetic was 0.25 g/m².

### (4) Sheet applying step

The sheet obtained in the electrostatic spraying step (2) was softly placed on the thin layer formed in the cosmetic applying step (3), and gently pressed.

### Evaluation

With regard to the coatings formed in the examples and comparative examples, the adhesion to the skin, and the waterproof properties and non-stickiness were evaluated as follows.

### 1. Evaluation of Adhesion

The evaluation was performed by visually observing the state of the coating after a microvibration load was applied by touching the coating with a finger in a direction orthogonal to the skin and a shearing force was applied to the coating by moving a finger back and forth in a direction parallel to the skin. Table 2 shows the results. The following are the evaluation criteria.
1 When a microvibration load is applied by a finger in the orthogonal direction, almost all the coating comes off.
2 When a microvibration load is applied by a finger in the orthogonal direction, a portion of the fibers included in the coating comes off.
3 The coating does not come off in the orthogonal direction, but when a shearing force is applied in the parallel direction, almost all the coating comes off.
4 The coating does not come off in the orthogonal direction, but when a shearing force is applied by a finger in the parallel direction, a portion of the fibers or coating comes off.
5 The coating does not come off in the orthogonal direction, and even when shearing force is applied in the parallel direction, the coating or the fibers do not come off.

### 2. Evaluation of Waterproof Properties

The evaluation was performed by running tap water from a faucet at 100 ml/sec onto a coating formed on skin, drying off the coating with a towel, and visually observing the state of the coating. Five grade evaluation was performed as follows: the coating that does not come off at all and has the best waterproof properties was evaluated as 5, and the coating that completely comes off and has no waterproof properties was evaluated as 1.

### 3. Evaluation of Feeling of Stickiness

The following evaluation was performed within three minutes immediately after a coating was formed.

Professional panelists formed a coating on the inner side of his or her forearm, and evaluated the feeling of stickiness while sliding a finger at 100 g/cm² over the formed coating. Five grade evaluation was performed regarding the feeling of stickiness as follows: the coating that is not sticky at all and has the best usability was evaluated as 5, and the coating that is sticky and is unpleasant to use was evaluated as 1. Table 2 shows the evaluation results obtained as averages of evaluation results from the three professional panelists.

**Table 1**

| | | Chemical name | Product name | Manufacturer | Cosmetic product 1 (mass%) | Cosmetic product 2 (mass%) | Cosmetic product 3 (mass%) | Cosmetic product 4 (mass%) | Cosmetic product 5 (mass%) | Cosmetic product 6 (mass%) | Cosmetic product 7 (mass%) |
|---|---|---|---|---|---|---|---|---|---|---|---|
| Oil phase | Solid oil (20°C) | Vaseline | Super White Protopet S1 | Sonneborn, LLC | 0 | 0 | 0 | 10 | 75 | 75 | 100 |
| | | Cetyl alcohol | Cetanol NX | Kokyu Alcohol Kogyo Co., Ltd. | 10 | | 30 | 6 | | 1.8 | |
| | | Stearyl alcohol | Stearyl alcohol NX | Kokyu Alcohol Kogyo Co., Ltd. | | 35 | 20 | 4 | | 1.2 | |
| | | Cetyl-PG hydroxyethyl palmitamide | Sphingolipid E | Kao Corporation | | | | 3 | | | |
| | Liquid oil | Neopentyl glycol dicaprate | Estemol N-01 | the Nisshin OilliO Group, Ltd. | | | | | 0.1 | | |
| | Polyol | Glycerin | Glycerin | Kao Corporation | 6.88 | 6.88 | 6.88 | 4.3 | 6.88 | 6.88 | |
| | Antiseptic | Phenoxy ethanol | Hisolve EPH | Toho Chemical Industry Co., Ltd. | 0.3 | 0.3 | 0.3 | 0.3 | 0.3 | 3 | |
| | Surfactant | PEG-60 hydrogenated castor oil | Emanon CH-60 | Kao Corporation | 1.6 | 1.6 | 1.6 | 0.4 | 0.4 | 0.4 | |
| | | Disodium stearoyl glutamate | Amisoft HS-21 | Ajinomoto Co., Inc. | | | | | 3 | | |
| Water phase | | 1,3 Butylene glycol | 1,3 Butylene glycol | Daicel Corporation | 3 | 3 | 3 | 8 | 3 | 3 | 0 |
| | | Water | Water | Kao Corporation | 78.22 | 53.22 | 38.22 | 64 | 11.32 | 8.72 | 0 |

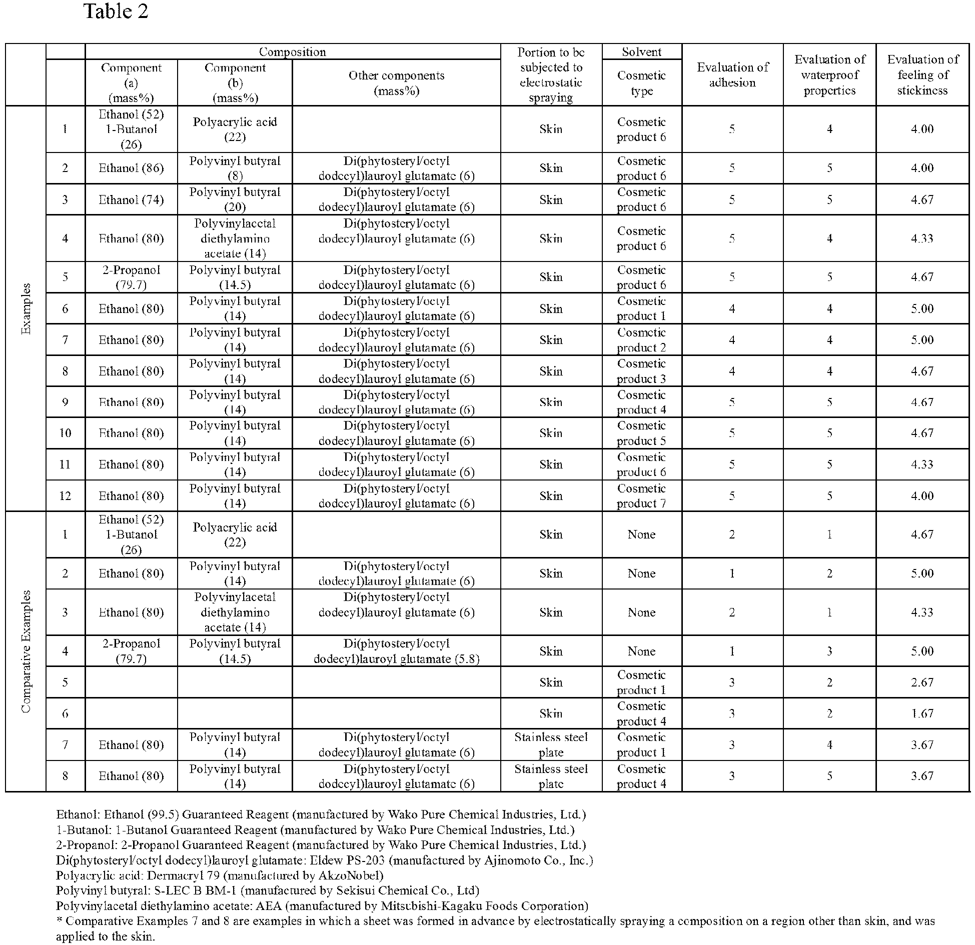

As is clear from the results shown in Table 2, it is found that the coatings formed with the methods of the examples have a higher adhesion to the skin and better waterproof properties, and are less sticky than those of the coatings formed with the methods of the comparative examples. Although not shown in the table, the color (white color) of the fibers was visually confirmed in the coatings formed with the methods of the comparative examples when the coatings were visually observed, whereas the transparency was confirmed in the coatings formed with the methods of the examples.

As is clear from the results shown in Table 2, it is found that coatings that have a high adhesion to the skin and good waterproof properties, and are not sticky were formed even when the types of oils and polyols used for the cosmetic were changed.

### Industrial Applicability

As described above in detail, according to the present invention, the adhesion between skin and a coating for a cosmetic formed through electrostatic spraying can be increased. Furthermore, the waterproof properties of a cosmetic covered by the coating can be increased, and a non-sticky coating for a cosmetic can be formed.

## Claims

1. A method for forming a coating for a cosmetic on a surface of skin, comprising:
a cosmetic applying step of applying a cosmetic containing 10 mass% or more of an oil, that is in a solid form at 20°C, onto skin; and
an electrostatic spraying step of electrostatically spraying a composition directly onto the skin, thereby forming a coating,
wherein the composition contains a component (a) and a component (b) below:
(a) one or more of volatile substances selected from the group consisting of water, alcohols, and ketones; and
(b) a polymer having a coating formation ability.

2. The method for forming a coating for a cosmetic according to claim 1, wherein in the electrostatic spraying step, the composition is electrostatically sprayed on the skin to form a porous coating.

3. The method for forming a coating for a cosmetic according to claim 1 or 2, wherein in the electrostatic spraying step, an electrostatic spraying apparatus is used to electrostatically spray the composition on the skin to form a porous coating composed of a deposit of fibers, and
the electrostatic spraying apparatus includes:
a container in which the composition is accommodated;
a nozzle from which the composition is discharged;
a supply device that supplies the composition accommodated in the container to the nozzle; and
a power source that applies a voltage to the nozzle.

4. The method for forming a coating for a cosmetic according to any one of claims 1 to 3, wherein in the cosmetic applying step, the cosmetic are dabbed onto the skin.

## Patentansprüche

1. Verfahren zur Bildung einer Beschichtung für ein Kosmetikum auf einer Hautoberfläche, umfassend:
einen Kosmetikum-Auftragungsschritt des Auftragens eines Kosmetikums, enthaltend 10 Massen-% oder mehr eines Öls, das bei 20°C in fester Form vorliegt, auf die Haut; und
einen elektrostatischen Sprühschritt des elektrostatischen Aufsprühens einer Zusammensetzung direkt auf die Haut, wodurch eine Beschichtung gebildet wird,
wobei die Zusammensetzung eine nachstehende Komponente (a) und eine nachstehende Komponente (b) enthält:
(a) eine oder mehrere flüchtige Substanzen, ausgewählt aus der Gruppe, bestehend aus Wasser, Alkoholen und Ketonen; und
(b) ein Polymer, das eine Beschichtungsbildungsfähigkeit aufweist.

2. Verfahren zur Bildung einer Beschichtung für ein Kosmetikum gemäß Anspruch 1, bei dem in dem elektrostatischen Sprühschritt die Zusammensetzung elektrostatisch auf die Haut gesprüht wird, um eine poröse Beschichtung zu bilden.

3. Verfahren zur Bildung einer Beschichtung für ein Kosmetikum gemäß Anspruch 1 oder 2, bei dem in dem elektrostatischen Sprühschritt eine elektrostatische Sprühvorrichtung verwendet wird, um die Zusammensetzung elektrostatisch auf die Haut zu sprühen, um eine poröse Beschichtung zu bilden, die aus einer Ablagerung von Fasern besteht, und
wobei die elektrostatische Sprühvorrichtung enthält:
einen Behälter, in dem die Zusammensetzung untergebracht ist;
eine Düse, aus der die Zusammensetzung ausgestoßen wird;
eine Versorgungsvorrichtung, die die in dem Behälter untergebrachte Zusammensetzung der Düse zuführt; und
eine Stromquelle, die eine Spannung an die Düse anlegt.

4. Verfahren zur Herstellung einer Beschichtung für ein Kosmetikum gemäß einem der Ansprüche 1 bis 3, bei dem in dem Kosmetikum-Auftragungsschritt das Kosmetikum auf die Haut aufgetupft wird.

## Revendications

1. Procédé de formation d'un revêtement pour un cosmétique sur une surface cutanée, comprenant :
une étape d'application de cosmétique pour appliquer un cosmétique contenant 10 % en masse ou plus d'une huile, qui est dans une forme solide à 20°C, sur la peau ; et
une étape de pulvérisation électrostatique pour pulvériser de manière électrostatique une composition directement sur la peau, formant ainsi un revêtement,
dans lequel la composition contient un composant (a) et un composant (b) ci-dessous :
a) une ou plusieurs substances volatiles choisies dans le groupe constitué de l'eau, d'alcools et de cétones ; et
b) un polymère ayant une capacité de formation de revêtement.

2. Procédé de formation d'un revêtement pour un cosmétique selon la revendication 1, dans lequel à l'étape de pulvérisation électrostatique, la composition est pulvérisée de manière électrostatique sur la peau pour former un revêtement poreux.

3. Procédé de formation d'un revêtement pour un cosmétique selon la revendication 1 ou 2, dans lequel à l'étape de pulvérisation électrostatique, un appareil de pulvérisation électrostatique est utilisé pour pulvériser de manière électrostatique la composition sur la peau pour former un revêtement poreux composé d'un dépôt de fibres, et
l'appareil de pulvérisation électrostatique inclut :
un récipient dans lequel la composition est logée ;
une buse à partir de laquelle la composition est libérée ;
un dispositif de fourniture qui fournit la composition logée dans le récipient à la buse ; et
une source d'alimentation qui applique une tension à la buse.

4. Procédé de formation d'un revêtement pour un cosmétique selon l'une quelconque des revendications 1 à 3, dans lequel à l'étape d'application de cosmétique, le cosmétique est tamponné sur la peau.
